# EUROPEAN PATENT APPLICATION

(11) **EP 2 711 711 A1**
(43) Date of publication of application: **26.03.2014**
(21) Application number: 12185015.0
(22) Date of filing: 19.09.2012
(51) Int. Cl.: G01N 33/86

(54) **Method for determining the direct thrombin inhibitor concentration in a plasma sample**

(71) Applicant: Technoclone GmbH, 1230 Vienna (AT)
(72) Inventor: Wagner, Lieselotte, 76669 Bad Schönborn (DE)
(74) Representative: Gassner, Birgitta

(57) **Abstract**

The present invention describes a reliable assay for determining the direct thrombin inhibitor concentration in a plasma sample potentially containing indirect thrombin inhibitors. The assay is insensitive to heparin due to the presence of a cationic polymer, thus allows the determination of the accurate amount of the direct thrombin inhibitor.

## Description

The present invention describes a reliable assay for determining the direct thrombin inhibitor concentration in a plasma sample potentially containing indirect thrombin inhibitors. The assay is insensitive to heparin due to the presence of a cationic polymer, thus allows the determination of the accurate amount of the direct thrombin inhibitor.

### BACKGROUND OF THE INVENTION

In anticoagulation therapy, increasing use is made of novel direct coagulation inhibitors, in particular direct thrombin (factor IIa) inhibitors. Said novel coagulation inhibitors have the potential to supersede the indirect coagulation inhibitors used to date, especially heparin and derivatives thereof, which only develop their coagulation inhibiting action in the presence of cofactors such as antithrombin or heparin cofactor II.

In medicine, heparin is often employed as a therapeutic substance to reduce the coagulability of blood. The formation of thromboses and embolisms can thereby be prevented.

Heparin remains the most widely used indirect thrombin inhibitor in clinical practice despite its well-known limitations. Direct thrombin inhibitors (DTIs) are a class of medication that act as anticoagulants by directly inhibiting thrombin. Some are in clinical use, while others are still undergoing clinical development. Several members of the class are expected to replace heparin (and its derivatives) and warfarin in various clinical scenarios.

The thrombin time is a control parameter in DTI treatment. The coagulation time of citrate or oxalate plasma is measured after addition of a standardized amount of thrombin.

The Technoclot DTI assay is based on a modified thrombin time determination. A diluted plasma sample is mixed with normal plasma and clotting is initiated by addition of a defined amount of thrombin reagent. The measured clotting times are proportional to inhibitor concentrations and can be directly read off calibrator specific calibration curves. The method can be run manually as well as adapted on coagulation analyzers.

To exclude undesirable influences of heparin on the coagulation assay, heparin can be removed from the sample before carrying out the test. It is known that heparin may be removed from samples by treatment with ion exchangers or via protamine bonded to a carrier. Alternatively, a complex of protamine and serum albumin is added to the sample and the heparin containing precipitate formed after some time of incubation is then filtered off.

These processes are very time-consuming and adversely influence the samples in respect of their coagulation properties and thus the concentration of the DTI might be overestimated.

The present invention was thus based on the technical problem of discovering a possibility of reducing or eliminating heparin sensitivity in coagulation tests.

Surprisingly it has been found in the context of the present invention that the presence of a cationic polymer in a suitable concentration can modify the heparin sensitivity of the coagulation assay.

### SUMMARY OF THE INVENTION

The present invention thus relates to a method for specific determination of a direct thrombin inhibitor in a plasma sample by measuring the thrombin time.

Dabigatran etexilate is a new direct thrombin inhibitor which is administered orally for prevention of venous thromboembolism (VTE) in patients after orthopedic surgery. The increasing use of new oral direct thrombin inhibitors (DTI) such as dabigatran creates the need to measure these direct thrombin inhibitors in the clinical routine.

The present invention also provides a diagnostic test which may be a clotting assay based on the inhibition of a constant and defined concentration of thrombin. Clotting times measured are directly related to thrombin inhibitor concentrations.

Calibration of the assay may be performed using lyophilized dabigatran calibrators with assigned values.

The assay can be performed in automated systems using coagulation analyzers.

### BRIEF DESCRIPTION OF THE DRAWINGS

The figures show:
Fig. 1: Calibration curve for dabigatran. The dependence of the thrombin time on the dabigatran concentration is shown.
Fig. 2a: Measurement of dabigatran control low with addition of different concentrations of unfractionated heparin (UFH).
Fig. 2b: Measurement of dabigatran control low with addition of different concentrations of low molecular weight heparin (LMWH).
Fig. 3: Calibration curve for dabigatran. The dependence of the thrombin time on the dabigatran concentration is shown.
Fig. 4a: Measurement of dabigatran control low with addition of different concentrations of unfractionated heparin (UFH).
Fig. 4b: Measurement of dabigatran control low with addition of different concentrations of low molecular weight heparin (LMWH).
Fig. 5: Deviation of the concentration of dabigatran from the expected vaulues.
   Dabigatran values are within the accepted limit range of ±10% for all samples containing UFH or LMWH up to 1.2 U/mL using an assay wherein polybrene is added.

### DETAILED DESCRIPTION OF THE INVENTION

The present invention relates to a method for specific determination of a direct thrombin inhibitor in a plasma sample potentially containing indirect thrombin inhibitors by measuring the thrombin time comprising the steps of
a. mixing a normal plasma sample with a diluted plasma sample,
b. neutralizing heparin and/or LMWH by the addition of a cationic polymer,
c. measuring the thrombin time, and
d. determining the amount of the direct thrombin inhibitor prolonging the thrombin time compared to a reference.

In a further aspect of the invention, the cationic polymer is added in a concentration of 0.1 to 20 mg/L, preferably in the range of 1 to 15 mg/mL, more preferred of 2 - 10 mg/L.

In a further aspect of the invention as described above, 4.5 mg/L of the cationic polymer are added.

In a further aspect of the invention as described above, the cationic polymer is polybrene.

In a further aspect of the invention as described above, the cationic polymer is added to the thrombin reagent.

In a further aspect of the invention as described above, the assay is used without adding metal salts.

The metals of the metal salt are preferably chosen from group 1 or group 2 of the Periodic Table of the Elements, such as for example copper or zinc.

In a further aspect of the invention as described above, the reference for determining the DTI concentration is a calibration curve.

In a further aspect of the invention as described above, a diagnostic test is provided which may be used in monitoring anticoagulant therapy.

In a further aspect of the invention is an assay kit for carrying out the method as described above at least containing a first reagent which contains lyophilized normal plasma and a second reagent which contains lyophilized thrombin and the cationic polymer, preferably polybrene.

The term "direct thrombin inhibitor" relates to non-physiological, preferably therapeutically active, substances which reduce the activity of a coagulation factor by direct interaction with said coagulation factor. Direct inhibitors of coagulation factors, for the determination of which the method according to the invention is suitable, are in particular direct inhibitors of the coagulation factor thrombin, for example dabigatran, argatroban, hirudin, melagatran, ximelagatran, bivalirudin, lepirudin, refludan, sofigatran, and AZD0837.

In a further aspect of the invention, the thrombin inhibitor is selected from the group consisting of dabigatran, argatroban, hirudin, melagatran, ximelagatran, bivalirudin, lepirudin, refludan, sofigatran, and AZD0837.

In this context, cationic polymers may include, e.g., DEAE-dextran, poly-L-lysine, modified polyaminoacids, such as β-aminoacid-polymers, or reversed polyamides, modified polyethylenes, such as poly(N-ethyl-4-vinylpyridinium bromide) (PVP), modified acrylates, such as poly(dimethylaminoethyl methylacrylate) (pDMAEMA), modified amidoamines, such as poly(amidoamine) (pAMAM), modified polybetaaminoesters (PBAE), such as diamine end modified 1,4 butanediol diacrylate-co-5-amino-1-pentanol polymers, dendrimers, such as polypropylamine dendrimers or pAMAM based dendrimers, polyimine, such as poly(ethyleneimine) (PEI), or poly(propyleneimine), polyallylamine, sugar backbone based polymers, such as cyclodextrin based polymers, cationic polysaccharides, for example chitosan, dextran, modified dextranes, hydroxypropylcellulose or polybrene, salicylamide derivatives such as polymedix, and protamine sulfate.

In the context of the present invention, a method is also disclosed for reducing the effect of heparin in an assay, in which a cationic polymer such as for example polybrene (hexadimethrine bromide) is either incorporated into at least one reagent of the test or separately added to the test.

Plasma samples obtained from blood drawn through heparinized tunneled venous access devices may contain heparin in unknown concentration. This heparin interferes with coagulation assays (Hinds et al., ONF 2002 29(3):E26-E34).

Direct thrombin inhibitors should be distinguished from "indirect thrombin inhibitors", which reduce the activity of a coagulation factor only by interaction with physiological cofactors, for example antithrombin III or heparin cofactor II. Indirect inhibitors of coagulation factors, to which the method according to the invention is insensitive, are in particular heparins and heparin-like substances, for example unfractionated high-molecular-weight heparins (UFHs), fractionated low-molecular-weight heparins (LMWHs), heparin derivatives and heparinoids. Low-molecular-weight heparin (LMWH) is a class of medication used as an anticoagulant in diseases that feature thrombosis, as well as for prophylaxis in situations that lead to a high risk of thrombosis. LMWHs, in contrast to UFH, consist of only short chains of polysaccharide. LMWHs are defined as heparin salts having an average molecular weight of less than 8000 Da and for which at least 60% of all chains have a molecular weight less than 8000 Da. These are obtained by various methods of fractionation or depolymerisation of polymeric heparin. Heparin derivatives and heparinoids are enzymatically and/or chemically modified glycosaminoglycan chains whose anticoagulatory properties may be biotechnologically modified by the specific manipulation of glycan chain length, sulfation or acetylation pattern or the mixture of different glycosaminoglycans. Synthetic heparins, such as the pentasaccharide fondaparinux, also have an indirect effect via antithrombin. For the purposes of the present invention, the term "heparin" comprehends hereinafter all aforementioned heparins, heparinoids and heparin derivatives.

Venous access devices are important in the management of advanced hematologic diseases. Several designs, including tunneled and nontunneled devices, are available. Long-term maintenance of devices consists of periodic flushes, typically with heparinized saline, to promote patency. Thus, plasma samples obtained from blood drawn through heparinized tunneled venous access devices may contain heparin in unknown concentration. Contamination of patient samples with heparin has a direct influence on the result of the coagulation assay. The thrombin time is prolonged due to heparin contamination. The thrombin time is also the basis for determining the direct thrombin inhibitor concentration such as for example the concentration of dabigatran. Thus calculated amount of the DTI is affected.

As an illustration of the present invention, several examples are provided below. These examples serve only to further illustrate aspects of the invention and should not be construed as limiting the invention.

### EXAMPLE 1 - Coagulation Assay Without the Addition of Polybrene

9 parts of venous blood and 1 part of sodium citrate solution (0.11 mol/L) are mixed and centrifuged for 15 min at a RCF of at least 2500 g (corresponding to DIN 58905). The plasma is stored at room temperature.

To obtain reagent 1 (R1) lyophilized normal plasma is reconstituted in 2 mL distilled water. For reagent 2 (R2), 26 IU of lyophilized bovine thrombin and a stabilizer are reconstituted in 2 mL distilled water.

DTI calibrator, control and patient sample are diluted 1:5 with 0.9% saline solution. 0.10 mL R1 is added to 0.05 mL of the diluted calibrator, control and patient plasma and the mixture is incubated at room temperatur for 4 min. Clotting is then initiated by adding the thrombin reagent R2. The clotting time measured is directly related to the concentration of the assayed DTI in the patient plasma sample.

**Calibration for Dabigatran**

| Coasys Plus C: C160293 | | | | |
|---|---|---|---|---|
| µg/mL | sec | | Mw | CV |
| 0,01 | 19,2 | 19 | **19,1** | 0,74% |
| 0,046 | 24,1 | 23,5 | **23,8** | 1,78% |
| 0,146 | 37,4 | 38,7 | **38,1** | 2,42% |
| 0,29 | 61,9 | 62,9 | **62,4** | 1,13% |
| 0,455 | 93,1 | 95,9 | **94,5** | 2,10% |

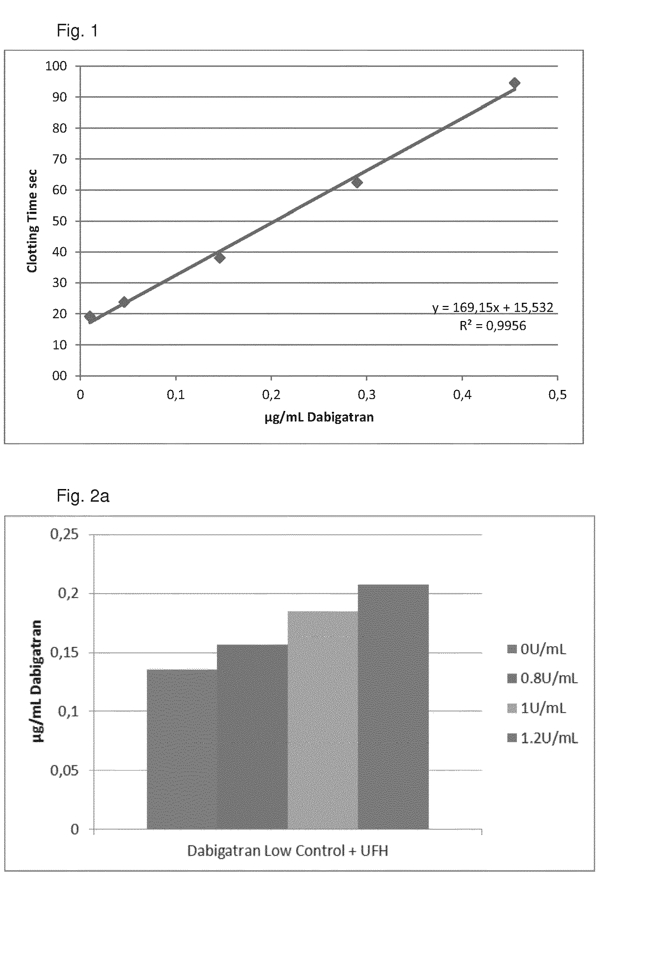

The calibration curve is depicted in Fig. 1.

**Measurement of dabigatran controls**

| | **Expected Value µg/mL** | **sec** | | **Mw** | **µg/mL** | | **Mw** |
|---|---|---|---|---|---|---|---|
| Dabigatran High Control | 0,273 | 59,6 | 64,4 | **62** | 0,275 | 0,301 | **0,288** |
| Dabigatran Low Control | 0,136 | 36,1 | 36,5 | **36,3** | 0,134 | 0,136 | **0,135** |

**Measurement of dabigatran Control Low with addition of different concentrations of Heparin**

| Dabigatran Control with | Mean (sec) | Mean (µg/mL) | Deviation from Control Value |
|---|---|---|---|
| UFH 1,2 U/ml | 48,1 | 0,2075 | -54% |
| UFH 1,0 U/ml | 44,2 | 0,1845 | -37% |
| UFH 0,8 U/ml | 39,7 | 0,1565 | -16% |
| LMW 1,2 U/ml | 43,3 | 0,179 | -33% |
| LMW 1,0 U/ml | 40,8 | 0,1635 | -21% |
| LMW 0,8U/ml | 39,0 | 0,152 | -13% |
| 0 U/mL | 36,3 | 0,135 | 0% |

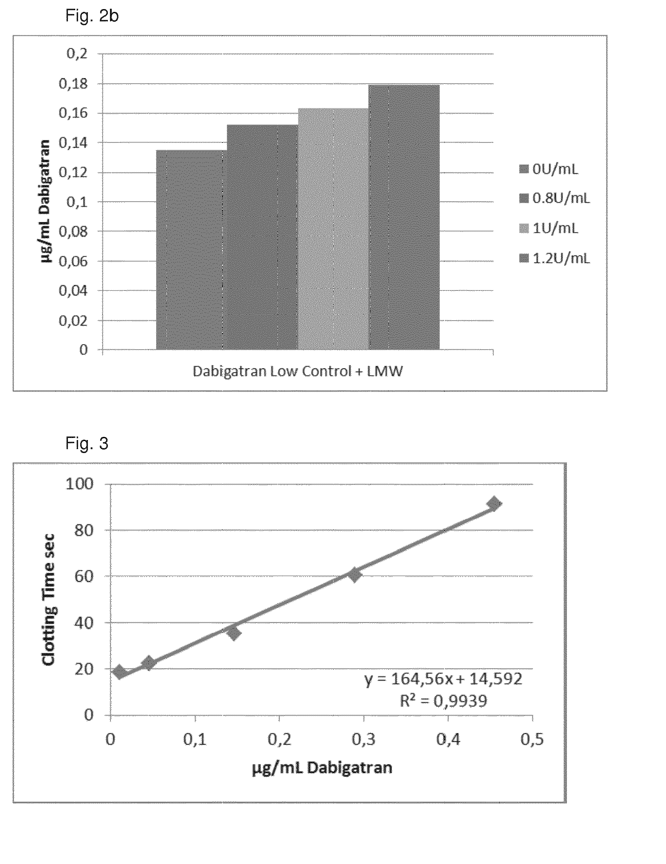

Fig. 2a and Fig. 2b show that the calculated amount of dabigatran is higher when compared to control. UFH and LMWH prolong the measured thrombin time and result in a higher amount of dabigatran.

### EXAMPLE 2 - Coagulation Assay With Addition of Polybrene

To the thrombin reagent R2 20 µg (10 mg/L) polybrene are added. Otherwise, the coagulation assay is carried out as described for example 1.

**Calibration for dabigatran**

| Coasys Plus C: C160293 | | | | |
|---|---|---|---|---|
| µg/mL | sec | | Mean | CV |
| 0,01 | 18,1 | 19,2 | **18,65** | 4,17% |
| 0,046 | 23,4 | 21,8 | **22,6** | 5,01% |
| 0,146 | 34,6 | 36,2 | **35,4** | 3,20% |
| 0,29 | 61,1 | 60,4 | **60,75** | 0,81% |
| 0,455 | 92,5 | 90,3 | **91,4** | 1,70% |

The calibration curve is depicted in Fig. 3.

**Measurement of dabigatran controls**

| | **Expected Value µg/mL** | **sec** | | **Mw** | **µg/mL** | | **Mw** |
|---|---|---|---|---|---|---|---|
| Dabigatran High Control | 0,273 | 58,3 | 59,7 | **59** | 0,277 | 0,284 | **0,281** |
| Dabigatran Low Control | 0,136 | 36 | 35,7 | **35,85** | 0,15 | 0,148 | **0,149** |

**Measurement of Dabigatran Control Low with addition of different concentrations of Heparin**

| Dabigatran Control with | **Mean (sec)** | **Mean (µg/mL)** | **Deviation from Control Value** |
|---|---|---|---|
| UFH 1,2 U/ml | 36,85 | 0,155 | 4,0% |
| UFH 1,0 U/ml | 34,85 | 0,1425 | -4,4% |
| UFH 0,8 U/ml | 34,95 | 0,143 | -4,0% |
| LMWH 1,2 U/ml | 38,1 | 0,1625 | 9,1% |
| LMWH 1,0 U/ml | 36 | 0,1495 | 0,3% |
| LMWH 0,8 U/ml | 37,15 | 0,157 | 5,4% |
| 0U/ml | 35,85 | 0,149 | 0% |

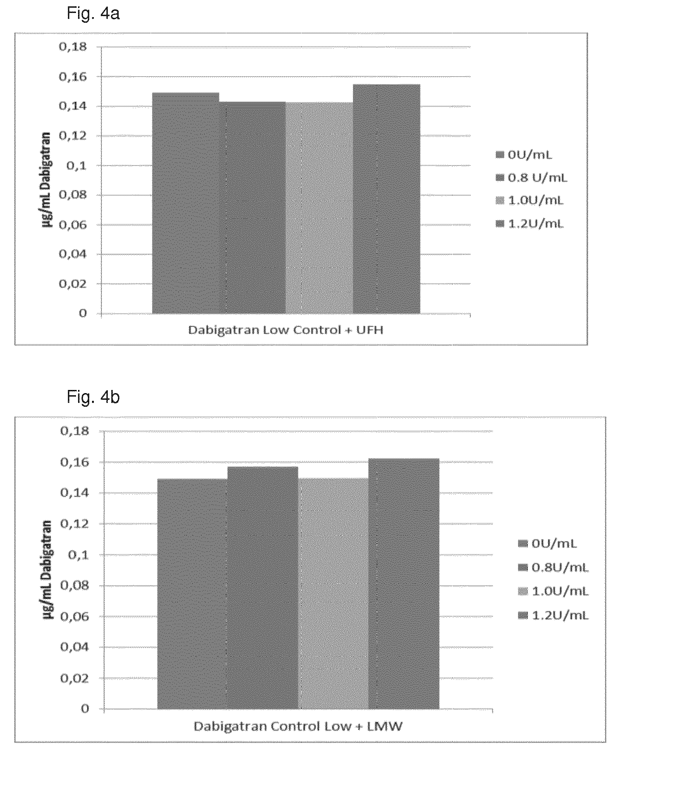

Fig. 4a and Fig. 4b show that the calculated amount of dabigatran correspond to control. Polybrene forms a complex with heparin and therefore the thrombin time is not prolonged and therefore the calculated amount of dabigatran is reliable.

### Conclusion

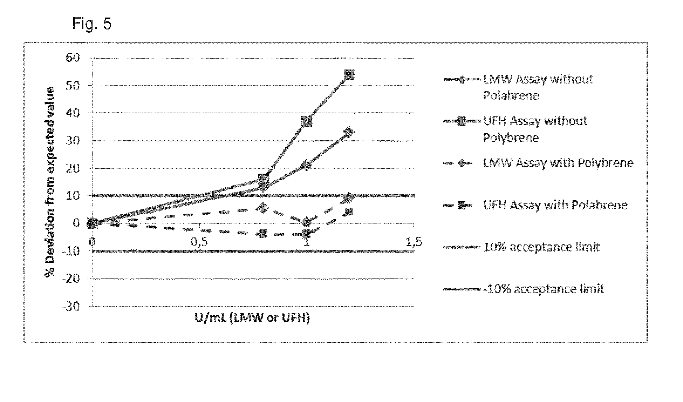

Using the assay without addition of polybrene in samples containing unfractionated Heparin (UFH) or Low Molecular Weight Heparin (LMWH) the Dabigatran concentration is overestimated. Dabigatran values are within the accepted limit range of ±10% for all samples containing UFH or LMWH up to 1.2 U/mL when the assay with addition of polybrene is used (see Fig. 5).

## Claims

1. A method for specific determination of a direct thrombin inhibitor in a plasma sample potentially containing indirect thrombin inhibitors by measuring the thrombin time comprising the steps of
a. mixing normal plasma with a diluted plasma sample,
b. neutralizing heparin and/or LMWH by the addition of a cationic polymer,
c. measuring the thrombin time, and
d. determining the amount of the direct thrombin inhibitor prolonging the thrombin time compared to a reference.

2. The method according to claim 1, wherein the cationic polymer is added in a concentration of 0.1 to 20 mg/L, preferably in the range of 1 to 15 mg/mL, more preferred 2 - 10 mg/L.

3. The method according to claim 2, wherein 4.5 mg/L of the cationic polymer are added.

4. The method according to any one of claims 1 to 3, wherein the cationic polymer is polybrene.

5. The method according to claim 1 to 4, wherein the cationic polymer is added to the thrombin reagent.

6. The method according to any one of claims 1 to 5, wherein the assay is used free of metal salts.

7. The method according to any one of claims 1 to 6, wherein the reference is a calibration curve.

8. The method according to any one of claims 1 to 7 for use in monitoring anticoagulant therapy.

9. The method according to any one of claims 1 to 8, wherein the direct thrombin inhibitor is selected from the group consisting of dabigatran, argatroban, hirudin, melagatran, ximelagatran, bivalirudin, lepirudin, refludan, sofigatran, and AZD0837.

10. An assay kit for carrying out a method according to any one of claims 1 to 9, at least containing a first reagent which contains lyophilized normal plasma and a second reagent which contains lyophilized thrombin and the cationic polymer, preferably polybrene.
